(19) Europäisches Patentamt
European Patent Office
Office européen des brevets

(11) Veröffentlichungsnummer: **0 197 274**
**B1**

(12) # EUROPÄISCHE PATENTSCHRIFT

(45) Veröffentlichungstag der Patentschrift: **04.01.89**

(51) Int. Cl.⁴: **C 07 C 1/32**, C 07 C 17/00, C 07 C 41/18, C 07 B 35/06

(21) Anmeldenummer: **86102371.1**

(22) Anmeldetag: **24.02.86**

(54) **Verfahren zur Herstellung von substituierten Styrolen.**

(30) Priorität: **06.03.85 DE 3507824**

(43) Veröffentlichungstag der Anmeldung: **15.10.86 Patentblatt 86/42**

(45) Bekanntmachung des Hinweises auf die Patenterteilung: **04.01.89 Patentblatt 89/1**

(84) Benannte Vertragsstaaten: **DE FR GB**

(56) Entgegenhaltungen:
**TETRAHEDRON LETTERS, Nr. 5, 1968, Seiten 613-616, Pergamon Press, Oxford, GB; A. NOVELLI et al.: "A new synthesis of trans-stilbenes"**

(73) Patentinhaber: **BAYER AG, Konzernverwaltung RP Patentabteilung, D-5090 Leverkusen 1 Bayerwerk (DE)**

(72) Erfinder: **Henneke, Karl- Wilhelm, Dr., Heymannstrasse 38, D-5090 Leverkusen 1 (DE)**
Erfinder: **Wedemeyer, Karlfried, Dr., Bilharzstrasse 7, D-5000 Köln 80 (DE)**

**EP 0 197 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von substituierten Styrolen aus substituierten N-Acyl-β-phenethylaminen.

Entsprechend der großen Bedeutung von substituierten Styrolen in der präparativen und technischen Chemie gibt es zahlreiche Methoden zu ihrer Herstellung. Die Verfahren zur gezielten Herstellung reiner, isomerenfreier, substituierter Styrole sind jedoch, soweit vorhanden. vom wirtschaftlichen Standpunkt aus gesehen häufig nicht optimal.

So ist bekannt, daß man aus Ethylbenzolen durch katalytische Dehydrierung Styrole herstellen kann (DE-OS-2 317 525). Nachteilig bei diesem Verfahren ist der meist unvollständige Umsatz des Ausgangsmaterials und damit verbunden eine aufwendige Destillation. Darüber hinaus sind substituierte Ethylbenzole, wie o-Chlorethylbenzol, in technischen Mengen nicht leicht verfügbar. Die Dehydrierungen sind darüber hinaus nur in Spezialapparaturen durchzuführen.

Bekannt ist die Herstellung von substituierten Styrolen in zweistufiger Reaktion ausgehend von substituierten Benzaldehyden. Nach Umsetzung mit Grignard-Reagentien erhält man α-Hydroxyalkylbenzole, die sauer dehydratisiert verden können [US-PS-2 404 319; Org. Synth., Coll. Vol III, 204 (1955)]. Nachteilig bei diesem Verfahren sind der hohe technische Aufwand, unter dem Grignard-Reaktionen durchgeführt werden müssen, die unbefriedigende Ausbeute und der häufig hohe Preis der Ausgangsaldehyde. Setzt man die Aldehyde nach Perkin mit Acetanhydrid um und decarboxyliert die entstandenen Zimtsäuren, so entstehen die substituierten Styrole meist nur in unbefriedigenden Ausbeuten [Ind. Eng. Chem. 50, 1005 (1958); Org. Synth., Coll. Vol. IV, 731 (1963)].

Es ist weiter bekannt, daß man aus substituierten Toluolen mit Methylhalogeniden oberhalb von 700°C Styrole herstellen kann (US-PS-3 636 182). Nachteilig bei diesem nur in Spezialapparaturen durchführbaren Verfahren sind der unvollständige Umsatz, die geringe Selektivität und ein hoher Aufwand zur Herstellung der Reinsubstanzen.

Man kann substituierte Styrole auch aus Benzylchloriden nach Wittig in zweistufiger Reaktion mit Triphenylphosphin und Formaldehyd herstellen (vgl. Houben-Weyl, Bd. 5/1b, S. 383 ff. ). Nachteilig bei diesem Verfahren sind der notwendige Einsatz von äquimolaren Menge eines teuren Hilfsreagenzes und die Entsorgung großer Mengen Triphenylphosphinoxids, was die Wirtschaftlichkeit des Verfahrens einschränkt.

Einige N-Acetyl-β-aminoalkylbenzole spalten in siedendem Xylol in Gegenwart von Phosphorpentoxid Acetamid ab und liefern z. B. die mittlere Doppelbindung in Stilbenen. Nachteilig bei diesen Synthesen sind der Einsatz mindestens molarer Mengen an Phosphorpentoxid und die unbefriedigenden Ausbeuten (Soc. 1949, 1074). Mittlere Doppelbindungen in Stilbenen können auch durch Abspalten von Acetamid in Gegenwart von Salzsäure hergestellt werden (Tetrahedron Letters 1968, 613). Allerdings dürfte diese Methode auf die Herstellung besonders aktivierter Stilbene beschränkt bleiben.

Es ist weiter bekannt, α-Alkylstyrole aus α-funktionalisierten Alkylbenzolen herzustellen (EP-110 536). Nachteilig bei dieser Methode ist, daß die Eliminierung von z. B. Wasser aus α-Hydroxyalkylbenzolen nicht einheitlich verläuft. Hierbei entstehen sowohl Styrole mit endständiger Doppelbindung in der Alkylkette als auch Styrole mit der Doppelbindung in 2-Stellung in der Alkylkette.

Es wurde nun ein Verfahren zur Herstellung von substituierten Styrolen der allgemeinen Formel

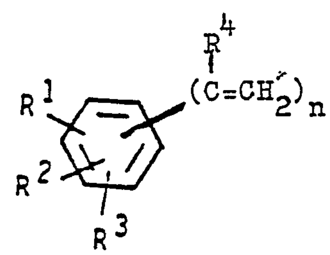

(I)

gefunden, worin

| | |
|---|---|
| $R^1$ bis $R^3$ | gleich oder verschieden sind und für Wasserstoff, Halogen, gegebenenfalls durch Halogen substituiertes niederes Alkyl oder niederes Alkoxy stehen, |
| $R^4$ | Wasserstoff oder niederes Alkyl bedeutet und |
| n | für 1 oder 2 steht, |

das dadurch gekennzeichnet ist, daß man N-Acyl-β-phenethylamine der allgemeinen Formel

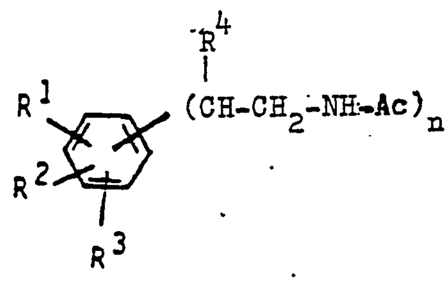

(II),

worin

Ac für einen Acylrest einer aliphatischen oder aromatischen Carbonsäure steht und

$R^1$ bis $R^4$ sowie n die oben angegebene Bedeutung haben,

mit Basen behandelt und während der Reaktion das gebildete Styrol abdestilliert.

Als niedere Alkylreste kommen solche mit 1 bis 5, bevorzugt 1 bis 4 Kohlenstoffatomen in Frage, wie der Methyl-, Ethyl-, n-Propyl-, iso-Propyl-, n-Butyl-, iso-Butyl-, sec.-Butyl- und tert.-Butyl-Rest, besonders bevorzugt der Methyl-, Ethyl- und iso-Propyl-Rest; als niedere Alkoxyreste solche mit 1 bis 5, bevorzugt 1 bis 3 Kohlenstoffatomen, wie der Methoxy-, Ethoxy-, Propoxy- und Isopropoxy-Rest. Als Beispiel für Halogensubstituierte Alkyl- oder Alkoxygruppen seien genannt: die Trifluormethyl- und Trifluormethoxygruppe.

Als Halogene seien genannt: Fluor, Chlor, Brom und Iod, bevorzugt Fluor und Chlor.

Nach dem erfindungsgemäßen Verfahren kann man z. B. herstellen:

2-Chlorstyrol, 3-Chlorstyrol, 4-Chlorstyrol, 3,4-Dichlorstyrol, 2,4-Dichlorstyrol, α-Methyl-3-chlorstyrol, α-Ethyl-3-chlorstyrol, α-Isopropyl-3-chlorstyrol, α-Butyl-3-chlorstyrol, α-Methyl-4-chlorstyrol, α-Ethyl-4-chlorstyrol, α-Isopropyl-4-chlorstyrol, α-Butyl-4-chlorstyrol, α-Methyl-3,4-dichlorstyrol, α-Ethyl-3,4-dichlorstyrol, α-Isopropyl-3,4-dichlorstyrol, α-Butyl-3,4-dichlorstyrol, 2,6-Dichlorstyrol, 2-Bromstyrol, 2,4-Dibromstyrol, 3-Bromstyrol, 4-Bromstyrol, 2-Fluorstyrol, 3-Fluorstyrol, 4-Fluorstyrol, α-Methyl-4-fluorstyrol, α-Methyl-4-fluorstyrol, α-Isopropyl-4-fluorstyrol, α-n-Butyl-4-fluorstyrol, 2-Methylstyrol, 3-Methylstyrol, 4-Methylstyrol, 2-Methoxystyrol, 4-Methoxystyrol, 2-Trifluormethylstyrol, 3-Trifluormethylstyrol, 4-Trifluormethoxystyrol, p-Divinylbenzol, m-Divinylbenzol und o-Divinylbenzol.

Der Acylrest des Ausgangsmaterials (siehe Formel (II)) leitet sich von Carbonsäuren oder Carbonsäurederivaten ab, die als Acylierungsmittel für Amine leicht und preiswert verfügbar sind; so z. B. der Acetyl-, Propionyl-, Isobutyryl- und der Benzoyl-Rest. Nach dem erfindungsgemäßen Verfahren werden bevorzugt die N-Acetyl-β-phenethylamine, wie sie sich aus der Formel (II) ergeben, eingesetzt.

Nach dem erfindungsgemäßen Verfahren kommen als Basen alle Verbindungen in Frage, die in der Lage sind, im chemischen Gleichgewicht die N-Acylamino-Funktion ins Anion zu überführen. Das können Alkoholate sein, wie Natriummethylat, Natriumethylat, Natriumisobutylat und/oder Kalium-tert.-butylat, bevorzugt Natriummethylat und/oder Kalium-tert.-butylat.

Weiterhin sind geeignet, die Alkalisalze von primären und sekundären Aminen, Ammoniak sowie Alkalihydride. Beispielsweise seien genannt: Natriumamid, Lithiumdiisopropylamid und/oder Natriumhydrid.

Es ist auch möglich, die Cyanide, Carbonate und/oder Fluoride der Alkali- und/oder Erdalkalimetalle einzusetzen, wie Natriumcarbonat, Kaliumcarbonat, Natriumhydrogencarbonat, Kaliumfluorid, Natriumfluorid und/oder Calciumcarbonat. Dabei ist es gegebenenfalls von Vorteil, wenn zusätzlich Phasentransferkatalysatoren hinzugefügt werden, die in der Lage sind, die Löslichkeit der Salze in der organischen Phase zu erhöhen; beim Kaliumion z. B. Kronenether, wie 18-Krone-6, oder Polyglykole.

Die Menge der nach dem erfindungsgemäßen Verfahren eingesetzten Basen kann in weiten Bereichen variiert werden. Im allgemeinen setzt man 0,1 bis 100 Mol-%, bevorzugt 1 bis 50 Mol-%, besonders bevorzugt 2 bis 10 Mol-%, bezogen auf N-Acyl-β-phenethylamin der allgemeinen Formel (II) ein. Die optimale Menge kann man aus Vorversuchen leicht ermitteln.

Nach dem erfindungsgemäßen Verfahren wird während der Reaktion das gebildete Styrol abdestilliert.

Die Reaktionstemperatur hängt im allgemeinen von der Konstitution des eingesetzten N-Acyl-β-phenethylamins ab. Die Temperatur beträgt gewöhnlich 150 bis 250°C, bevorzugt 160 bis 200°C.

Der Zusatz eines Lösungs- und/oder Verdünnungsmittels ist im Prinzip nicht erforderlich und in den meisten Fallen auch überflüssig. Bei Einsatz von höherschmelzen den Ausgangsmaterialien kann die Verwendung von Lösungs- und/oder Verdünnungsmitteln gegebenenfalls von Vorteil sein. Als inerte Lösungs- und/oder Verdünnungsmittel kommen aliphatische und/oder aromatische Kohlenwasserstoffe, aliphatische und/oder aromatische Ether sowie halogenierte aromatische Kohlenwasserstoffe in Frage. Ihre Siedepunkte können so hoch sein, daß sie unter den Destillationsbedingungen im Sumpf verbleiben. Es ist aber gegebenenfalls von Vorteil, ihren Siedepunkt so zu wählen, daß die Lösungs und/oder Verdünnungsmittel unter den Reaktions- und Destillationsbedingungen ganz oder teilweise mit überdestillieren. Polymerisationsfreudige Styrole, wie Divinylbenzole, werden so verdünnt und schneller aus den heißeren Apparateteilen verdrängt. Als Beispiele für geeignete Lösungs- und/oder Verdünnungsmittel seien genannt: Anthracen, Phenanthren, Terphenyl,

Polyglykolether, Polyglykole, Phenoxydiphenyle und/oder isomere Tolylether.

Setzt man N-Acetyl-2-(2-chlorphenyl)-ethylamin und Natriummethylat als Ausgangsstoffe in das erfindungsgemäße Verfahren ein, so kann der Reaktionsablauf durch folgende Formelgleichung wiedergegeben werden:

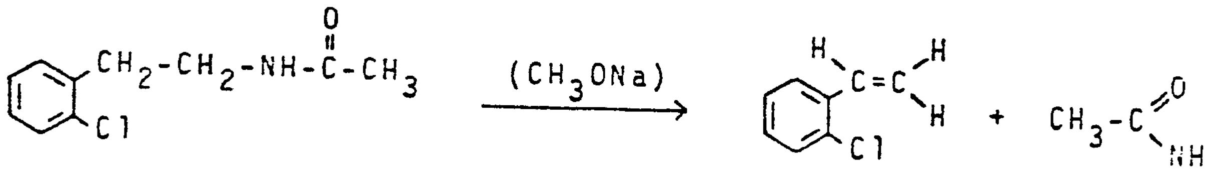


Das erfidungsgemäße Verfahren kann man z. B. wie folgt in einer gewöhnlichen Destillationsapparatur durchführen. Die Apparatur besteht aus einem Reaktionskolben mit aufgesetzter 10 bis 20 cm Vigreux-Kolonne und darüber einem Luftkühler, der die Produkte in eine kühlbare Vorlage leitet. Man legt das N-Acyl-β-phenethylamin der Formel (II), 2 bis 10 Mol-% Alkoholat und etwa 0,1 Gew.-% tert.-Butylbrenzcatechin vor und gibt die gleiche Menge an Stabilisator in die Destillationsvorlage. Nach Anlegen von 10 bis 20 mbar Vakuum erhitzt man auf etwa 150 bis 250°C. Dabei destillieren die Spaltprodukte über. In der Vorlage kristallisiert Acetamid aus dem Destillat meist vollständig aus. Es kann durch Filtration oder durch Waschen mit Wasser vom Styrol getrennt werden. Nach Auswaschen evtl. in geringer Menge vorhandener basischer Bestandteile mit verdünnter Salzssäure erhält man die Styrole durch Redestillation über eine Brücke oder eine kurze Kolonne in hoher Reinheit.

Auf die Zugabe eines Polymerisation-Stabilisators kann gegebenenfalls verzichtet werden, insbesondere bei den Styrolen der allgemeinen Formel (I), in denen $R^4$ eine Alkylgruppe ist.

Man kann das erfindungsgemäße Verfahren auch kontinuierlich durchführen, indem man in einer kleineren Apparatur einen Teil des N-Acetyl-β-phenethylamins vorlegt und je nach Reaktions- und Destillationsfortschritt weiteres Ausgangsmaterial zudosiert.

Die in dem erfindungsgemäßen Verfahren eingesetzten substituierten N-Acyl-β-phenethylamine sind in an sich bekannter Weise aus den entsprechend substituierten Benzylchloriden leicht herzustellen (vgl. Herstellungsbeispiele). Wie aus dem Formelschema ersichtlich, erfolgt zunächst ein Chlor/Cyan-Austausch. Die Benzylcyanide sind in α-Stellung alkylierbar. Nach Hydrierung zu den β-Phenethylaminen und folgender Acylierung erhält man das Ausgangsmaterial der allgemeinen Formel (II).

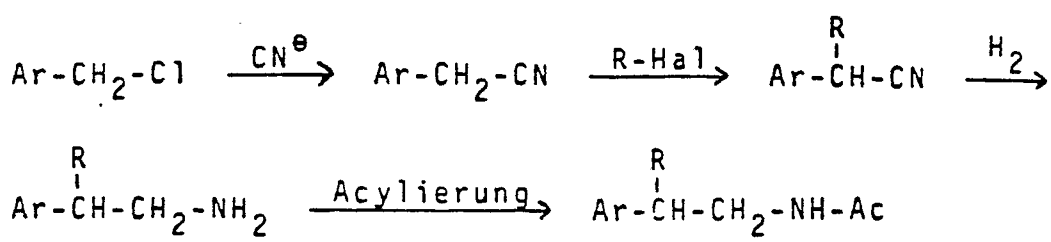


Das erfindungsgemäße Verfahren hat mehrere Vorteile gegenüber dem Stand der Technik. Aus gut verfügbaren reinen Benzylchloriden sind in technisch einfach durchzuführenden Reaktionsschritten in üblichen Apparaturen reine, isomerenfreie Styrole in hoher Ausbeute und Qualität herstellbar. Bei den seitenkettenalkylierten Derivaten entstehen ausschließlich Produkte mit endständiger Doppelbindung.

Substituierte Styrole finden vielfältige Anwendung in der Polymer- und Wirkstoffchemie. Bei der Copolymerisation dienen sie zur Modifizierung von Produkteigenschaften (siehe Ullmanns Encyclopädie der technischen Chemie, 4. Auflage, Band 22, Seite 306 ff.). Auf Basis substituierter Styrole sind in jüngerer Zeit neue Wirkstoffe beschrieben die als Pflanzenschutzmittel eingesetzt werden können.

Die nachfolgenden Beispiele sollen das erfindungsgemäße Verfahren verdeutlichen.

**Beispiel 1**

Eine Vakuum-Destillationsapparatur, bestehend aus einem 500 ml-Dreihalskolben mit Innenthermometer, 10 cm Vigreux-Kolonne, aufgesetzter Brücke und Vorlage, wird mit Stickstoff gespült. Man gibt 197,5 g (1 Mol) N-Acetyl-2-(2-chlorphenyl)ethylamin, 5,9 g (50 mmol) Kalium-tert.-butylat (95-%-ig) und 0,2 g tert.-Butylbrenzcatechin in den Kolben und 0,2 g des gleichen Stabilisators in die Vorlage. Nach Einstellen eines Druckes von 20 mbar wird das Ausgangsmaterial erhitzt. Ab ca. 160°C Sumpftemperatur beginnt die Reaktion, erkennbar am Erscheinen der Spaltprodukte im Kühler. Man führt die Reaktion in 1 bis 2 h durch Erwärmen bis zu einer Sumpftemperatur von 200°C zu Ende. Das Vakuum wird zuletzt kurzfristig auf ca. 5 mbar erhöht. Man erhält 188,3 g Destillat, aus dem Acetamid weitgehend auskristallisiert. Das Destillat wird in 300 ml Ether und 200 ml Wasser aufgenommen, die wäßrige Phase wird abgetrennt, die organische Phase nacheinander mit 5-

%-iger Salzsäure und wäßriger Natriumhydrogencarbonat-Lösung gewaschen. Nach Trocknen und Abdestillieren des Lösungsmittels erhält man 133,3 g Rückstand, der 99,1 % o-Chlorstyrol enthält. Ausbeute 95,4 % der Theorie.

**Beispiele 2 bis 7**

Man verfährt wie unter Beispiel 1 beschrieben, setzt aber die in der Tabelle angegebenen Basen ein. Destillationssümpfe wurden bis 220° C ausgeheizt.

| Beispiel | Base | Mol-%[1] | Ausbeute % d.Th. | Selektivität[2] % |
|---|---|---|---|---|
| 2 | Natriummethylat | 4 | 94 | 95 |
| 3 | Kaliumisobutylat | 5 | 94 | 95 |
| 4 | NaCN | 10 | 32 | 84 |
| 5 | KCN[3] | 5 | 90 | 95 |
| 6 | $K_2CO_3$[3] | 2,5 | 86 | 92 |
| 7 | KF[3] | 5 | 45 | 78 |

1) bezogen auf N-Acetyl-2-(2-chlorphenyl)ethylamin
2) bezogen auf umgesetztes Einsatzmaterial
3) zusätzlich 18-Krone-6 zugegeben. Menge äquivalent zu Kaliumionen.

**Beispiele 8 bis 25**

Man verfährt wie unter Beispiel 1 beschrieben und erhält die substituierten Styrole der Tabelle aus den entsprechenden N-acetyl-Vorstufen der allgemeinen Formel (II).

| Beispiel | 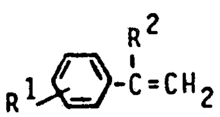 R¹ | R² | K-tert.-butylat[1] Mol-% | Spaltungstemperatur[2] °C | Destillationsdruck mbar | Styrolausbeute[3] % d.Th. | Selektivität[4] % |
|---|---|---|---|---|---|---|---|
| 8 | 4-Cl | H | 5 | 170-185 | 20 | 91 | 92 |
| 9 | 2,4-$Cl_2$ | H | 10 | 160-180 | 10 | 73 | 91 |
| 10 | 3,4-$Cl_2$ | H | 10 | 160-180 | 10 | 85 | 89 |
| 11 | 4-Cl | $(CH_3)_2CH$ | 10 | 180-190 | 10 | 62 | 86 |
| 12 | 4-Cl | $(CH_3)_2CH$ | 10[5] | 180-190 | 10 | 82 | 92 |
| 13 | 4-Cl | $CH_3$-$CH_2$ | 25 | 180-190 | 10 | 83 | 93 |
| 14 | 3,4-$Cl_2$ | $(CH_3)_2CH$ | 25 | 170-195 | 10 | 80 | 89 |
| 15 | 4-$CH_3$ | H | 40 | 185-200 | 20 | 64 | 81 |
| 16 | 4-$CH_3$ | H | 10[5] | 180-205 | 20 | 81 | 95 |
| 17 | 4-F | H | 10 | 170-190 | 20 | 85 | 89 |
| 18 | 2-F | H | 20 | 170-200 | 20 | 40 | 67 |
| 19 | 4-Br | H | 5 | 180-205 | 10 | 29 | 88 |
| 20 | 4-Br | H | 10[5] | 175-210 | 10 | 73 | 91 |
| 21 | 2-Br | H | 10[5] | 180-190 | 10 | 87 | 88 |
| 22 | 3-$CF_3$ | H | 20 | 165-200 | 20 | 80 | 84 |
| 23 | 2-$CF_3$ | H | 30 | 165-180 | 20 | 50 | 72 |
| 24 | 4-$CF_3O$ | H | 20 | 175-200 | 10 | 64 | 80 |
| 25 | 4-$CH_3O$ | H | 40 | 190-250 | 10 | 45 | 47 |

1) Bezogen auf Acetylphenethylamin der Formel (II)
2) Richtwerte
3) im Destillat
4) Styrolausbeute bezogen auf umgesetztes Acetylphenylethylamin. Nicht umgesetztes Ausgangsmaterial befinde sich teils im Destillat, teils im Destillationsrückstand.
5) Zusätzlich 18-Krone-6 zugegeben. Menge ist äquimolar zur Basemenge.

**Beispiel 26**

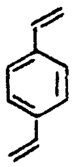

In einer Destillationsapparatur analog Beispiel 1 werden unter $N_2$ 248,3 g (1 Mol) 1,4-Bis-(2-acetaminoethyl)-benzol, 11,2 g (100 mmol) Kalium-tert.-butylat 95-%-ig), 26,4 g (100 mmol) 1,4,7,10,13,16-Hexaoxacyclooctadecan (18-Krone-6), 250 g eines Gemisches isomerer Phenoxydiphenylether, 250 mg tert.-Butylbrenzcatechin und 250 mg p-Phenylendiamin vorgelegt. In die Destillationsvorlage gibt man zusätzlich 250 mg tert.-Butyl-brenzcatechin. Nach Anlegen eines Vakuums von 10 mbar erwärmt man den Sumpf. Ab ca. 175°C beginnt die Spaltreaktion, erkennbar am Erscheinen der Produkte in der Destillationsbrücke. Bei langsam ansteigender Sumpftemperatur bis auf 210°C führt man die Reaktion in 1 - 2 h zu Ende. Zuletzt wird das Vakuum kurzfristig auf 5 mbar erhöht. Man nimmt das Destillat in 300 ml Ether und 200 ml Wasser auf, trennt die wäßrige Phase ab, wäscht die organische Phase noch einmal mit 200 ml Wasser, trocknet mit Natriumsulfat und destilliert das Lösungsmittel im Vakuum ab. 213,2 g Rückstand enthält 58,4 % p-Divinylbenzol (96 % d.Th.). 2,6 % 4-(2-Acetaminoethyl)-styrol und 37,3 % isomere Phenoxydiphenylether. Nach erneuter Destillation über eine 20 cm Vigreux-Kolonne erhält man mit Siedepunkt 40 - 41°C bei ca. 0,5 mbar 111,0 g p-Divinylbenzol. Ausbeute 85 % d. Th. Reinheit 99,9 %.

**Beispiel 27**

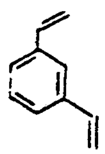

Man setzt 248,3 g (1 Mol) 1,3-Bis-(2-acetaminoethyl)-benzol ein und verfährt wie unter Beispiel 26 beschrieben. 191,8 g Etherrückstand enthält 58,2 % m-Divinylbenzol (86 % d.Th. ), 10,0 % 3-(2-Acetaminoethyl)-styrol und 30,1 % isomere Phenoxydiphenylether. Nach Destillation über eine 20 cm-Vigreux-Kolonne erhält man 104,5 g m-Divinylbenzol. Ausbeute 80 % d.Th. Reinheit 99,6 %.

**Beispiel 28**

Man verfährt zunächst wie unter Beispiel 1 beschrieben. Wenn die Spaltprodukte überdestillieren, wird aus einem Tropftrichter ein weiteres Mol N-Acetyl-2-(2-chlorphenyl)-ethylamin in den Reaktionskolben zugetropft. Die Dosiergeschwindigkeit wird so geregelt, daß die zugetropfte Menge etwa der Destillatmenge entspricht. Die Basenmenge beträgt 2,5 Mol-% bezogen auf die insgesamt eingesetzte Acetylverbindung.

Nach Aufarbeitung erhält man 274,4 g Etherrückstand, der 97,2 % = 266,6 g = 96 % d.Th. o-Chlorstyrol enthält.

**Beispiel 29**

Man setzt 259,7 g (1 Mol) N-Benzoyl-2-(2-chlorphenyl)-ethylamin ein und verfährt sonst wie bei Beispiel 1.

Bei bis zu 200°C Sumpftemperatur und 140°C Kopftemperatur bei 20 mbar erhält man 73,6 g Destillat, das 82,6 % = 60,8 g = 44 % d.Th. o-Chlorstyrol enthält. 188 g Destillationsrückstand enthalten 134,2 g Ausgangsverbindung. Selektivität 91 %.

**Beispiel 30**

Man verfährt wie unter Beispiel 1 beschrieben, ersetzt aber Kalium-tert.-butylat durch 5 g gepulvertes Kaliumcarbonat und 100 g Polyglykol P 400. Der Sumpf wird bis 215° C ausgeheizt. Destillat bis zu 140° C/6 mbar abgenommen. 120,2 g Etherrückstand nach Aufarbeitung enthält 96,9 % o-Chlorstyrol. Ausbeute 84 % d.Th.

**Herstellung des Ausgangsmaterials**

<u>a) 4-Clor/Cyan-Austausch</u>

4-Chlorbenzylchlorid

In einer 2-l-Mehrhalsapparatur mit Rückflußkühler, Innenthermometer und Tropftrichter werden 196 g (4 Mol) Natriumcyanid, 12,5 g (40 mmol) Tributylbenzylammoniumchlorid und 660 ml Wasser vorgelegt und auf 90° C erhitzt. Man tropft bei gleicher Temperatur in 1 h 644 g (4 Mol) geschmolzenes 4-Chlorbenzylchlorid hinzu und rührt noch 2 h nach. Nach Abkühlen auf ca. 35° C wird die organische Phase abgetrennt, mit Wasser gewaschen und über eine kurze Kolonne fraktioniert. Man erhält 552 g 4-Chlorbenzylcyanid (91 % d.Th.)

<u>b) Seitenkettenalkylierung</u>

4-Chlor-α-isopropyl-benzylcyanid

In einer 4-l-Mehrhalsapparatur mit Rückflußkühler und Innenthermometer werden 469 g (3,1 Mol) 4-Chlorbenzylcyanid, 30 g Tetrabutylammoniumbromid, 1500 g Natronlauge 50-%-ig und 438 g (3,56 Mol) Isopropylbromid vorgelegt. Nach Anstellen des Rührers steigt die Temperatur an. Sie wird anfangs durch Kühlen, später durch Heizen 4 h bei 40° C gehalten. Man kühlt auf Raumtemperatur, gießt das Reaktionsgemisch in eine Mischung aus 500 ml Ether und 500 ml Wasser, trennt die wäßrige Phase ab und wäscht die organische Phase nacheinander mit 5-%-iger Salzsäure, Natriumhydrogencarbonat-Lösung und Wasser. Nach Trocknen mit Natriumsulfat wird das Lösungsmittel abdestilliert. Als Rückstand verbleiben 585,3 g 97-%-iges 4-Chlor-α-isopropyl-benzylcyanid. Das Produkt ist für die Folgehydrierung rein genug.

<u>c) Hydrierung</u>

4-Chlor-α-isopropyl-β-phenethylamin

Ein 0,7-l-Autoklav wird mit 400 g (2 Mol) 97-%-igem 4-Chlor-α-isopropyl-benzylcyanid, 23 g methanolfeuchtem Raney-Cobalt und 80 g Ammoniak (4,7 Mol) gefüllt. Man erhöht den Gesamtdruck mit Wasserstoff auf 100 bar und erhitzt auf 130° C. Durch Nachdrücken von Wasserstoff auf 100 bis 150 bar ist die Reaktion in ca. 1 h zu Ende. Nach Abkühlen auf Raumtemperatur, Absaugen des Katalysators und Nachwaschen mit Methanol wird das Rohprodukt über eine Kolonne fraktioniert destilliert. Mit Siedepunkt 134 - 135°/16 mbar erhält man 352 g (89 % d.Th.) 4-Chlor-α-isopropyl-β-phenethylamin. Reinheit 99,8-%-ig.

<u>d) Acetylierung</u>

Unter Feuchtigkeitsausschluß tropft man zu vorgelegtem 208 g (2,04 Mol) Essigsäureanhydrid in 1 h bei 60° C 395,4 g (2 Mol) 4-Chlor-α-isopropyl-β-phenethylamin. Man rührt 2 h bei 60°C nach und destilliert das Reaktionsgemisch im steigenden Vakuum über eine Brücke. Nach Abtrennen der Essigsäure bzw. des überschüssigen Essigsäureanhydrid destillieren im Ölpumpenvakuum 460 g reines N-Acetyl-4-chlor-α-isopropyl-β-phenethylamin mit Sdp. 168 - 171° C/ca. 1 - 2 mbar.

Alle anderen N-Acetyl-β-phenethylamine der allgemeinen Formel (II) wurden in analoger Reaktionssequenz hergestellt. Bei festem Einsatzmaterial oder festen Produkten wurden Lösungsmittel eingesetzt, so Toluol für den Chlor/Cyan-Austausch und die Seitenkettenalkylierung, Methanol für die Hydrierung und Essigsäure für die Acetylierung.

**Patentansprüche**

1. Verfahren zur Herstellung von substituierten Styrolen der Formel

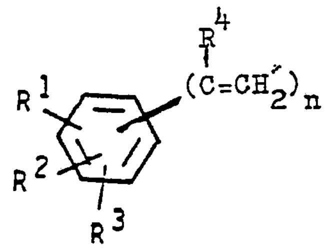

worin
$R^1$ bis $R^3$ gleich oder verschieden sind und für Wasserstoff, Halogen, gegebenenfalls durch Halogen substituiertes niederes Alkyl oder niederes Alkoxy stehen,
$R^4$ Wasserstoff oder niederes Alkyl bedeutet und
n für 1 oder 2 steht,
dadurch gekennzeichnet, daß man N-Acyl-β-phenethylamine der Formel

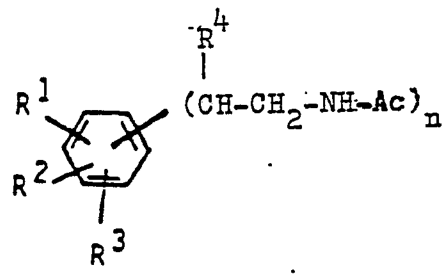

worin
Ac für einen Acylrest einer aliphatischen oder aromatischen Carbonsäure steht und
$R^1$ bis $R^4$ sowie n die oben angegebene Bedeutung haben,
mit Basen behandelt und während der Reaktion das gebildete Styrol abdestilliert.

2. Verfahren nach Anspruch 1, dadurch gekenneichnet, daß man N-Acetyl-β.'-phenethylamine einsetzt.

3. Verfahren nach Ansprüchen 1 und 2, dadurch gekennzeichnet, daß man als Basen Alkoholate, Alkalisalze von primären und sekundären Aminen, Ammoniak, Alkalihydride und/oder die Cyanide, Carbonate und/oder Fluoride der Alkali- und/oder Erdalkalimetalle einsetzt.

4. Verfahren nach Ansprüchen 1 bis 3, dadurch gekennzeichnet, daß man bei Einsatz von Basen, die Alkali- und/oder Erdalkaliionen enthalten, Phasentransferkatalysatoren zusetzt.

5. Verfahren nach Ansprüchen 1 bis 4, dadurch gekennzeichnet, daß man als Phasentransferkatalysatoren Kronenether oder Polyglykol zusetzt.

6. Verfahren nach Ansprüchen 1 bis 5, dadurch gekennzeichnet, daß man die Basen in Mengen von 0,1 bis 100 Mol-%, bezogen auf N-Acyl-β-phenethylamin einsetzt.

7. Verfahren nach Ansprüchen 1 bis 6, dadurch gekennzeichnet, daß man die Basenbehandlung bei Temperaturen von 150 bis 250°C durchführt.

**Claims**

1. Process for the preparation of substituted styrenes of the formula

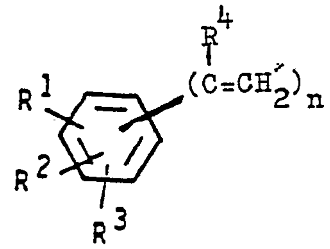

in which

| | |
|---|---|
| $R^1$ to $R^3$ | are identical or different and represent hydrogen, halogen, lower alkyl or lower alkoxy each of which is optionally substituted by halogen, |
| $R^4$ | denotes hydrogen or lower alkyl, and |
| n | represents 1 or 2, |

characterised in that N-acyl-β-phenethylamines of the formula

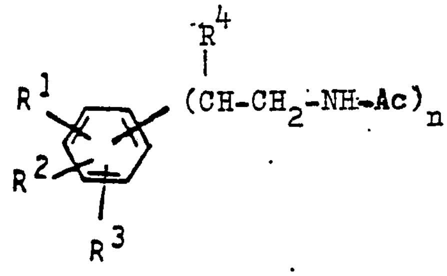

in which

| | |
|---|---|
| Ac | represents an acyl radical of an aliphatic or aromatic carboxylic acid, and |

$R^1$ to $R^4$ and n have the abovementioned meaning,
are treated with bases and the styrene which is formed is removed by distillation during the reaction.

2. Process according to Claim 1, characterised in that N-acetyl-β-phenethylamines are used.

3. Process according to Claims 1 and 2, characterised in that the bases used are alcoholates, alkali metal salts of primary and secondary amines, ammonia, alkali metal hydrides and/or the cyanides, carbonates and/or fluorides of the alkali metals and/or alkaline earth metals.

4. Process according to Claims 1 to 3, characterised in that phase-transfer catalysts are added when bases which contain alkali metal and/or alkaline earth metal ions are used.

5. Process according to Claims 1 to 4, characterised in that crown ethers or polyglycol are added as the phase-transfer catalysts.

6. Process according to Claims 1 to 5, characterised in that the bases are used in amounts of from 0.1 to 100 mol-% relative to N-acyl-β-phenethylamine.

7. Process according to Claims 1 to 6, characterised in that the base treatment is carried out at temperatures of from 150 to 250° C.

## Revendications

1. Procédé de production de styrènes substitués de formule

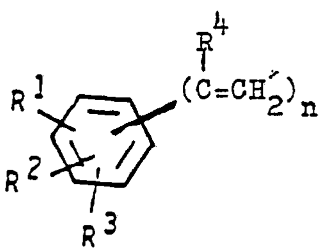

dans laquelle

| | |
|---|---|
| $R^1$ à $R^3$ | sont identiques ou différents et représentent l'hydrogène, un halogène, un groupe alkyle inférieur ou alkoxy inférieur éventuellement substitué par un halogène, |
| $R^4$ | représente l'hydrogène ou un groupe alkyle inférieur et |
| n | a la valeur 1 ou 2, |

caractérisé en ce qu'on traite avec des bases des N-acyl-β-phénéthylamine de formule

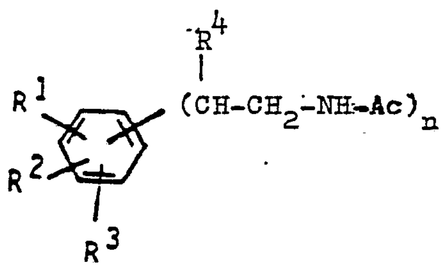

dans laquelle

Ac représente un reste acyle d'un acide carboxylique aliphatique ou aromatique et

$R^1$ à $R^4$ de même que n ont la définition indiquée ci-dessus, et on chasse par distillation le styrène formé pendant la réaction.

2. Procédé suivant la revendication 1, caractérisé en ce qu'on utilise des N-acétyl-β-phénéthylamines.

3. Procédé suivant les revendications 1 et 2, caractérisé en ce qu'on utilise comme bases des alcoolates, des sels de métaux alcalins d'amines primaires et secondaires, de l'ammoniac, des hydrures alcalins et/ou des cyanures, carbonates et/ou fluorures des métaux alcalins et/ou des métaux alcalino-terreux.

4. Procédé suivant les revendications 1 à 3, caractérisé en ce qu'on ajoute des catalyseurs de transfert de phase dans le cas de l'utilisation de bases qui contiennent des ions de métaux alcalins et/ou de métaux alcalino-terreux.

5. Procédé suivant les revendications 1 à 4, caractérisé en ce qu'on ajoute comme catalyseurs de transfert de phase des éthers en couronne ou un polyglycol.

6. Procédé suivant les revendications 1 à 5, caractérisé en ce qu'on utilise les bases en quantités de 0,1 à 100 moles %, par rapport à la N-acyl-β-phénéthylamine.

7. Procedé suivant les revendications 1 à 6, caractérisé en ce qu'on conduit le traitement avec des bases à des températures de 150 à 250°C.